(19) **Europäisches Patentamt / European Patent Office / Office européen des brevets**

(11) **EP 3 601 587 B1**

(12) **FASCICULE DE BREVET EUROPEEN**

(45) Date de publication et mention de la délivrance du brevet:
**06.01.2021 Bulletin 2021/01**

(21) Numéro de dépôt: **18722105.6**

(22) Date de dépôt: **29.03.2018**

(51) Int Cl.:
*C12Q 1/00* (2006.01)  *C12R 1/32* (2006.01)
*C12Q 1/04* (2006.01)  *C12N 1/20* (2006.01)
*C12Q 1/48* (2006.01)  *G01N 27/00* (2006.01)

(86) Numéro de dépôt international:
**PCT/FR2018/050769**

(87) Numéro de publication internationale:
**WO 2018/178578 (04.10.2018 Gazette 2018/40)**

(54) **MÉTHODE DE DÉTECTION ÉLECTROCHIMIQUE DE MYCOBACTÉRIES**

VERFAHREN ZUR ELEKTROCHEMISCHEN DETEKTION VON MYCOBAKTERIEN

METHOD FOR ELECTROCHEMICAL DETECTION OF MYCOBACTERIA

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.03.2017 FR 1752787**

(43) Date de publication de la demande:
**05.02.2020 Bulletin 2020/06**

(73) Titulaires:
• **Université de Bourgogne**
  **21000 Dijon (FR)**
• **Institut national de recherche pour l'agriculture, l'alimentation et l'environnement**
  **75007 Paris (FR)**

(72) Inventeurs:
• **ROCHELET, Murielle**
  **21000 Dijon (FR)**
• **BARBIER, Élodie**
  **21320 Rouvres-Sous-Meilly (FR)**
• **HARTMANN, Alain**
  **21000 Dijon (FR)**

(74) Mandataire: **Novagraaf Technologies**
**Bâtiment O2**
**2, rue Sarah Bernhardt**
**CS90017**
**92665 Asnières-sur-Seine Cedex (FR)**

(56) Documents cités:
• **PHUNPAE PONRUT ET AL: "Rapid diagnosis of tuberculosis by identification of Antigen 85 in mycobacterial culture system", DIAGNOSTIC MICROBIOLOGY AND INFECTIOUS DISEASE, vol. 78, no. 3, 7 décembre 2013 (2013-12-07), pages 242-248, XP028610595, ISSN: 0732-8893, DOI: 10.1016/J.DIAGMICROBIO.2013.11.028 cité dans la demande**
• **BOUCAU J ET AL: "A coupled assay measuring Mycobacterium tuberculosis antigen 85C enzymatic activity", ANALYTICAL BIOCHEMISTRY, ELSEVIER, AMSTERDAM, NL, vol. 385, no. 1, 21 octobre 2008 (2008-10-21), - 1 février 2009 (2009-02-01), pages 120-127, XP025838523, ISSN: 0003-2697, DOI: 10.1016/J.AB.2008.10.018 [extrait le 2008-10-21] cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

**Domaine de l'invention**

La présente invention a pour objet un procédé ou une méthode de détection des mycobactéries qui repose sur la mesure de l'activité acyltransférase, en particulier de l'activité catalytique de l'Antigène 85, avec une méthode d'analyse électrochimique.

La présente invention trouve ses applications en médecine humaine et vétérinaire, pour le diagnostic des mycobactérioses et de la tuberculose humaine et animale, ainsi qu'en diagnostic environnemental.

Dans la description ci-dessous, les références entre crochets ([ ]) renvoient à la liste des références présentée à la fin du texte.

**État de la technique**

Les mycobactéries appartiennent au Phylum des Actinobacteria et se caractérisent par une paroi riche en acides mycoliques leur conférant des propriétés tinctoriales particulières liées à la résistance de leur paroi aux décolorations successives par un acide puis par l'alcool à 90° (BAAR : Bacilles Acido-Alcolo Résistants). Environ 200 espèces appartenant au genre Mycobacterium ont été identifiées à ce jour.

Parmi elles, les mycobactéries dites tuberculeuses sont des bactéries pathogènes obligatoires à tropisme majoritairement respiratoire responsables de tuberculose chez l'homme et l'animal. Les mycobactéries non tuberculeuses (dites atypiques ou environnementales) regroupent des bactéries opportunistes à l'origine de mycobactérioses chez l'homme et les animaux. La lèpre est également une maladie causée par une mycobactérie, *Mycobacterium leprae*.

La tuberculose humaine est principalement due à *M. tuberculosis,* mais peut-être causée également par *M. africanum, M. canettii, M. bovis* notamment. La tuberculose humaine représente un problème de santé majeur sur le plan mondial car c'est la maladie infectieuse la plus meurtrière dans le monde (environ 1,5 millions de décès en 2013) avec le VIH (sida). L'OMS (Organisation Mondiale de la Santé) estime le nombre de nouveaux cas à environ 9 millions chaque année.

Concernant les animaux, la tuberculose touche de très nombreuses espèces : les bovins, les caprins ainsi que de nombreuses espèces sauvages comme les petits rongeurs, par exemple.

Les mycobactéries atypiques, largement retrouvées dans l'environnement (sol et eau), présentent une pathogénicité très variable chez l'homme et les animaux. Chez l'homme, l'incidence des infections liées aux mycobactéries atypiques semble augmenter dans les pays industrialisés. Elles surviennent généralement sur un terrain d'immunodépression locale ou générale provoquant majoritairement des infections pulmonaires (par exemple *M. avium et M. intracellulare, M. xenopi, M. kansasii, M. malmoense*), des infections ganglionnaires (*M. avium et intracellulare, M. kansasii, M. scorofulaceum*), cutanées (M. marinum, M. ulcerans, M. chelonae), voire systémiques (*M. avium et intracellulare, M. kansasii, M. haemophilum, M. xenopi, M. gevanense)...* Chez les animaux, certaines provoquent des infections contagieuses à forte morbidité et mortalité (paratuberculose chez les bovins : *M. avium ssp. paratuberculosis* et *« tuberculose » des oiseaux: M. avium ssp. avium* par exemple). La persistance des mycobactéries atypiques dans l'environnement, leur résistance aux détergents et l'aptitude de certaines espèces à former des biofilms, notamment dans les réseaux d'eau, peut être à l'origine de la contamination de l'eau de surface et des réseaux de distribution à l'origine de contamination humaine.

La détection des mycobactéries chez l'homme, l'animal ou dans l'environnement repose sur les techniques suivantes.

La méthode de détection historique repose sur un examen microscopique des échantillons (frottis d'expectoration, broyat de lésion...) qui permet de mettre en évidence la présence de Bacilles Acido-Alcoolo Résistants (BAAR), caractéristique partiellement spécifique des mycobactéries. Chez l'homme, l'examen microscopique est réalisé sur un frottis d'échantillon biologique ou le culot de centrifugation obtenu après fluidification-décontamination des produits pathologiques contaminés. Deux colorations sont utilisées : la coloration de Ziehl-Neelsen (microscopie classique) et la coloration à l'auramine (microscopie à fluorescence). C'est un examen clé puisque la majorité des cas de tuberculose dans les pays à forte incidence sont diagnostiqués de cette façon dans les centres périphériques de microscopie. L'examen microscopique reste également le point de départ du schéma diagnostique adopté dans les laboratoires de diagnostic des pays développés. L'examen microscopique est facile à mettre en œuvre (peu de matériel, personnel peu qualifié), avec un rendu de résultat rapide (2 à 3 heures) et un coût faible. Toutefois, cet examen présente plusieurs inconvénients : une mise en œuvre opérateur-dépendant, une interprétation subjective du résultat, un manque de sensibilité (détection de 50 à 70 % des cas de tuberculose pulmonaire), ainsi qu'une absence d'identification de l'espèce impliquée. Ses performances sont encore plus réduites chez les patients infectés par le VIH et les enfants (prélèvements paucibactériens).

Concernant la détection bactériologique de la tuberculose et des mycobactérioses chez l'homme et l'animal, ainsi que celle de la contamination environnementale, la technique de référence reste la culture sur milieu adapté (solide : Coletsos, Löwenstein-Jensen, Middlebrook 7H11 ou liquide : Middlebrook 7H9) éventuellement supplémenté en antibiotiques et antifongiques. La mise en culture de la bactérie à partir d'expectorations, de broyats tissulaires, d'autres échantillons biologiques ou de prélèvements environnementaux est couramment utilisée et a l'avantage d'être sensible. Des systèmes automatisés de culture liquide type Bactec MGIT™ (Becton Dickinson) ou BacT/Alert® (Bio Mérieux) associent l'incubation et la mesure spectroscopique de la croissance bactérienne. Toutefois, cette méthode ne permet qu'un diagnostic retardé puisque d'un point de vue microbiologique et cultural, les mycobactéries tuberculeuses et une partie des mycobactéries atypiques sont des microorganismes à croissance lente : 1 à 6 semaines minimum d'incubation à 37°C sont nécessaires pour observer la croissance de la bactérie sur les milieux de culture. Quelle que soit la nature de l'échantillon, un traitement préalable de décontamination associé ou non avec une fluidification avec des agents physico-chimiques (N-acétylcystéine - soude, hypochlorite de sodium, acides, détergents) est indispensable avant sa mise en culture pour prévenir le développement de microorganismes à croissance rapide, réduisant la sensibilité de la méthode. Suite à la culture, l'identification de l'espèce peut être réalisée par des méthodes d'hybridation de l'ADN éventuellement couplées à de la PCR, du séquençage de gènes, du génotypage (séquences d'insertion, spoligotypage...), par l'identification de biomarqueurs (analyse des acides mycoliques par chromatographie en phase liquide, profil protéique par spectrométrie de masse type MALDI-TOF par exemple). Cependant, toutes ces techniques nécessitent un appareillage de laboratoire coûteux et un personnel très qualifié et ne sont donc pas adaptées au diagnostic délocalisé et rapide des infections mycobactériennes.

En raison de leur rapidité (rendu du résultat dans la journée), les méthodes de biologie moléculaire sont également aujourd'hui largement utilisées dans les laboratoires de diagnostic tant pour l'homme que pour l'animal et l'environnement. Basées sur l'amplification spécifique de gènes mycobactériens cibles, elles permettent à la fois la détection de la bactérie et son identification, voire ses éventuelles résistances aux antibiotiques (diagnostic humain uniquement). Cependant, leur utilisation nécessite généralement des infrastructures adaptées, un appareillage coûteux ainsi que du personnel qualifié (extraction d'ADN et interprétation des résultats). La technologie GeneXpert® mise au point par le laboratoire Cepheid pour le diagnostic moléculaire de la tuberculose humaine limite les inconvénients cités précédemment grâce à l'utilisation d'un automate qui réalise toutes les étapes sans intervention humaine. Le résultat obtenu en 2 heures permet de détecter la présence d'une mycobactérie tuberculeuse ainsi que sa résistante potentielle à la rifampicine, un antibiotique de première ligne utilisé dans le traitement de la tuberculose humaine. Son prix constitue toutefois un frein à sa généralisation dans les pays à faibles revenus. De plus, cette technologie ne permet pas de faire plus d'une vingtaine d'analyses par jour.

Chez l'animal, le diagnostic de tuberculose est réalisé post-mortem après un dépistage prophylactique ou une découverte de lésions à l'abattoir. Il est réalisé sur des broyats de lésions ou de nœuds lymphatiques. Comme chez l'homme, les échantillons peuvent être mis en culture après décontamination et/ou être analysés par des méthodes de biologie moléculaire. Les limites de cette détection sont identiques à celles citées précédemment : rendu de résultat retardé avec la culture et automates coûteux de biologie moléculaire avec personnel qualifié.

Concernant la détection des mycobactéries dans l'environnement, leur recherche n'est pas standardisée et aucune norme n'est actuellement disponible. La culture sur milieu adapté après décontamination chimique se heurte aux mêmes limites que celle des échantillons biologiques : croissance lente avec rendu de résultat tardif et contamination des milieux de culture par des bactéries à croissance rapide notamment. La biologie moléculaire a permis de court-circuiter cette étape de culture, mais ne permet pas toujours une quantification adaptée.

En vue de proposer de nouvelles méthodes d'identification des mycobactéries, la détection d'antigènes spécifiques comme Ag MPT64 ou ceux du complexe de l'Antigène 85 (Ag85) ont été envisagées. Pour ce faire, un test immunochromatographique basé sur l'identification de l'Ag MPT64 après culture (présent chez les mycobactéries tuberculeuses, absent chez les atypiques) a été commercialisé (SD Bioline TB Ag MPT64, Standard Diagnostic, Inc.). Des tests ELISA utilisés pour la détection de l'Ag85 dans des filtrats de cultures liquides, du sérum ou dans du liquide cérébrospinal (Phunpae et al., Diagn. Microbiol. Infect. Dis., 78(3) : 242-248, 2014 [1]; Kashyap et al., BMC infectious diseases, 7 :74, 2007 [2]; Kashyap et al., Clin Diagn Lab Immunol., 12(6) :752-758, 2005 [3]) sont décrits dans la littérature.

Le complexe de l'Antigène 85 (Ag85) est composé de trois protéines homologues sécrétées : l'antigène 85A (Ag85A), l'antigène 85B (Ag85B), et l'antigène 85C (85C) qui partagent une identité de séquence élevée (68-79%) sous leurs formes matures sécrétées. Il s'agit de mycolyltransférases (enzymes de poids moléculaire d'environ 30 000 Da) qui interviennent spécifiquement dans la construction et le maintien des parois des Corynebacteriales - Ordre auquel appartient le genre *Mycobacterium* - en catalysant le transfert de l'acide mycolique sur des structures polysaccharidiques (arabinogalactane, tréhalose). Plus généralement, l'Ag85 est une acyltransférase qui est non seulement capable de transférer des groupements mycolyles mais également d'autres groupements acyles.

La détection et l'activité de l'Ag85 étant largement étudiées pour la recherche et l'évaluation de nouvelles méthodes de diagnostic et de suivi d'efficacité de traitements de chimiothérapie antituberculeuse (Elamin et al., J Microbiol Methods, 79(3) :672-678, 2002 [4]), plusieurs méthodes spectrophotométriques (UV-visible, fluorescence, *etc...*) ont été décrites pour le dosage de cette protéine *via* la mesure de son activité acyltransférase (Boucau et al., Analytical Biochem.,

385 : 120-127, 2009 [5] ; Favrot et al., J. Biol. Chem., 289(36) : 25031-25040, 2014 [6]).

La Demande internationale WO 2011/030160 [7] décrit une méthode pour détecter la présence de mycobactéries dans un organisme ou un échantillon biologique *via* la mise en évidence de l'activité catalytique de l'Ag85 pendant l'étape de culture. Pour ce faire, des sondes moléculaires constituées d'un polysaccharide (tréhalose et autres dérivés osidiques) marqué (radiotraceur, fluorophore, nanoparticules, biotine) ont été synthétisées et ajoutées au milieu de culture afin d'être incorporées dans la paroi de la bactérie au cours de la croissance bactérienne grâce à l'activité transférase de l'Ag85. A l'issue de cette étape, les bactéries sont rincées et isolées du milieu de culture puis détectées à l'aide d'une technique adaptée (compteur scintillation, fluorimètre, microscopie, RMN, techniques d'imagerie in vivo, etc...). Toutefois, cette méthode qui permet de la détection des mycobactéries viables en les marquant, ne peut être envisagée que pour l'analyse d'échantillons très contaminés (de l'ordre de $10^7$ bacilles.mL$^{-1}$) ou après une étape de culture assez longue. De plus, plusieurs étapes de rinçage des bactéries sont obligatoires pour éliminer l'excès de sonde marquée non incorporée. Enfin, la détection du marqueur est réalisée à l'aide de techniques instrumentales de laboratoire fragiles et coûteuses qui nécessitent du personnel qualifié pour leur mise en œuvre et l'interprétation des résultats.

La Demande de Brevet CN102087283 [8] décrit une méthode de détection électrochimique de *M. tuberculosis* à l'aide d'un immunocapteur enzymatique à base d'une solution de chitosane, de nanoparticules d'or et d'un anticorps spécifique de la paroi cellulaire de *M. tuberculosis.* La mesure quantitative est réalisée en comparant le signal du produit généré par la phosphatase alcaline en présence d'a-naphtyl phosphate avant et après incubation avec l'échantillon. Toutefois, bien que cette méthode ait été appliquée à la détection de *M. tuberculosis* dans des échantillons de lait, son utilisation en diagnostic de routine ne peut pas être envisagée. En effet, l'utilisation d'électrodes de carbone vitreux pour la construction de l'immunocapteur est très contraignante : polissage de la surface et nettoyage dans un mélange piranha (acide sulfurique et peroxyde d'hydrogène) avant chaque nouvelle utilisation. De plus, la préparation de la surface sensible de l'immunocapteur nécessite plusieurs étapes: 1) électrodéposition d'une solution contenant des nanoparticules d'or, du chitosane et un anticorps de chèvre anti-souris marqué avec une phosphatase alcaline puis 2) incubation d'une solution d'anticorps anti-*M. tuberculosis* produit chez la souris. Enfin, une fois construit, l'immunocapteur doit être conservé à 4°C.

Il existe donc un besoin d'une méthode de détection des mycobactéries simple et rapide à mettre en œuvre et palliant les inconvénients des procédés de l'art antérieur.

## Description de l'invention

Afin de répondre à ce besoin de test de diagnostic de la tuberculose humaine, animale et/ou environnementale plus performant pour lequel il n'existe à ce jour pas de solution adaptée, les inventeurs ont mis au point une nouvelle méthode électrochimique capable de détecter rapidement (obtention des résultats en environ 2 à 5 h) la présence ou l'absence de mycobactéries dans des échantillons comme par exemple dans un milieu de culture et dans des prélèvements respiratoires humains : la méthode EDMYC (Electrochemical Detection of MYCobacteria).

Ainsi, les inventeurs ont développé une nouvelle méthode de détection électrochimique des mycobactéries *via* la mesure électrochimique de l'activité acyltransférase dans les mycobactéries, en particulier de l'activité catalytique de l'Ag85 en présence d'un substrat de l'enzyme, *e.g.* le p-aminophényl-6-O-octanoyl-β-D-glucopyranoside (p-AP-OG), et d'un cofacteur ou co-substrat, *e.g.* du tréhalose. En effet, comme l'Ag85 est très largement excrété par les mycobactéries, *e.g.* par *Mycobacterium tuberculosis et Mycobacterium bovis,* dans les milieux de culture liquide, la détection de l'activité acyltransférase, notamment de l'activité catalytique de l'Ag85, dans le milieu de culture permet de mettre en évidence la présence de mycobactéries.

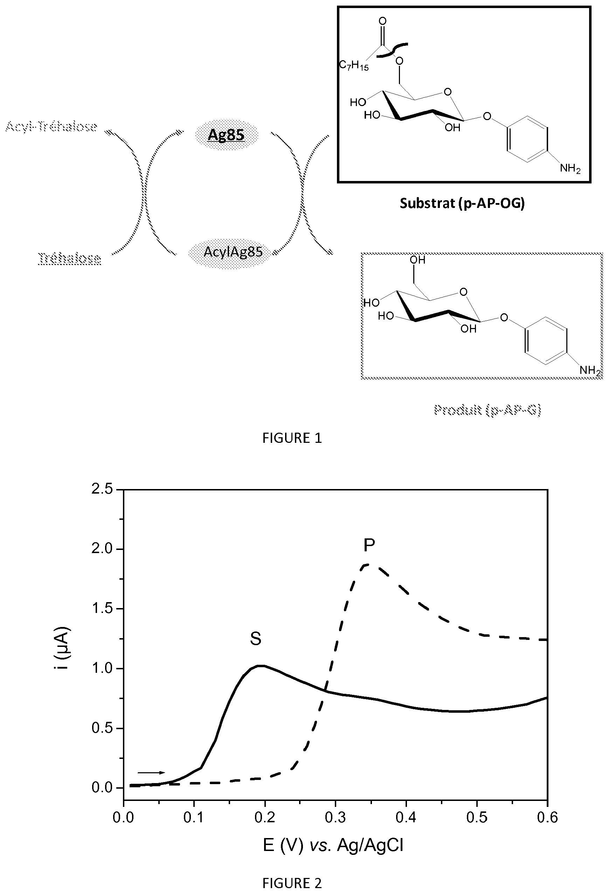

Le principe de l'invention repose sur le fait que les acyltransférases telles que l'Ag85 hydrolysent la liaison ester du substrat, et transfèrent le groupement acyle ainsi libéré sur le cofacteur. Le produit est alors détecté par voltammétrie. Selon un mode de réalisation particulier, la présente invention repose sur la capacité des acyltransférases, en particulier de l'Ag85, à hydrolyser la liaison ester du p-AP-OG et de transférer le groupement octanoyle du p-AP-OG sur un sucre, *e.g.* le tréhalose, selon un mécanisme ping-pong, pour respectivement générer le p-aminophényl-β-D-glucopyranoside (p-AP-G) et former l'acyl-tréhalose (figure 1). La différence entre les potentiels des pics d'oxydation du p-AP-OG et du p-AP-G observée sur les voltammogrammes (ou voltampérogrammes) (figure 2), qui s'explique respectivement par la présence ou l'absence du groupement octanoyle sur la molécule, rend donc possible la détection spécifique de l'activité acyltransférase, en particulier de l'activité catalytique de l'Ag85, en présence de p-AP-OG. L'intensité du pic d'oxydation du p-AP-G, choisie comme réponse analytique, est proportionnelle à la quantité d'acyltransférases, en particulier d'Ag85, et donc à celle des mycobactéries présentes dans l'échantillon analysé.

Ainsi, les inventeurs ont mis au point une méthode de détection simple et rapide des mycobactéries et de leur viabilité avec ou sans étape de culture préalable.

A ce jour, la preuve de concept de la méthode a été démontrée avec succès avec la détection de plusieurs espèces de mycobactéries fréquemment rencontrées dans des infections pulmonaires dont *M. tuberculosis* - l'agent principal de la tuberculose chez l'homme - dans des cultures liquides et solides. La méthode présente de nombreux avantages par rapport à l'examen microscopique comme sa simplicité de mise en œuvre ou encore une interprétation facile des résultats (mesure chiffrée) avec un appareillage de taille réduite, portable et peu coûteux. En outre, par rapport aux méthodes optiques, la technique électrochimique s'avère particulièrement avantageuse puisqu'elle permet l'analyse d'échantillons troubles ou colorés, avec la possibilité d'offrir une mesure quantifiée, avec une bonne sensibilité, à l'aide de capteurs sérigraphiés à usage unique. Ainsi, elle répond parfaitement au cahier des charges imposé par l'OMS pour un test capable par exemple de remplacer l'examen microscopique de frottis d'expectorations.

En outre, les Inventeurs ont mis en évidence une amélioration de la spécificité de la méthode de détection vis-à-vis des mycobactéries et de l'Ag85 en proposant 1) l'utilisation d'un substrat de l'enzyme avec des groupements acyles possédant des chaînes carbonées plus longues que celle du p-AP-OG, par exemple des chaînes alkyles allant de $C_7H_{15}$ à $C_{29}H_{59}$, et/ou 2) une méthode d'extraction et de décontamination des échantillons réels pour isoler les mycobactéries.

La présente invention a donc pour objet un procédé de détection électrochimique de mycobactéries dans un échantillon biologique, ledit procédé comprenant les étapes de :

a) sélection d'un substrat d'au moins une acyltransférase et de son cofacteur ;
b) mise en contact dudit échantillon biologique avec lesdits substrat et cofacteur ;
c) détection électrochimique du produit résultant de l'activité catalytique de ladite au moins une acyltransférase.

Selon un mode de réalisation particulier du procédé de détection de la présente invention, l'échantillon biologique est choisi dans le groupe constitué de : cultures bactériennes, prélèvements biologiques d'origine humaine ou animale, échantillons environnementaux, etc... Une culture bactérienne peut être par exemple obtenue sur une gélose nutritive ou dans un milieu de culture liquide, selon des techniques bien connues de l'Homme du métier. Un prélèvement biologique d'origine humaine peut être par exemple un échantillon d'origine pulmonaire (crachats, sécrétions bronchiques, biopsie), un échantillon de sang, un échantillon de liquide céphalorachidien, un échantillon d'urine, un échantillon d'origine intestinale (biopsie intestinale, fèces), ainsi que tout autre échantillon tissulaire. Un prélèvement biologique d'origine animale peut être par exemple un échantillon tissulaire (nœud lymphatique, poumon, foie, rate...), un échantillon de fèces ou un échantillon de lait. Un échantillon d'origine environnementale peut être par exemple un échantillon d'eaux usées, ou d'eaux usées hospitalières, un échantillon d'eaux usées traitées, un échantillon de boues issues du traitement des eaux usées ou un échantillon de sol.

Selon un mode de réalisation particulier du procédé de détection de la présente invention, le substrat d'acyltransférase est choisi dans le groupe constitué de : p-aminophényl-6-O-octanoyl-β-D-glucopyranoside, substrats avec des groupements acyles possédant des chaînes alkyles allant de $C_7H_{15}$ à $C_{29}H_{59}$.

Selon un mode de réalisation particulier du procédé de détection de la présente invention, le cofacteur est un sucre choisi dans le groupe constitué de : tréhalose, D-glucose.

De préférence le substrat est le p-aminophényl-6-O-octanoyl-β-D-glucopyranoside, et le cofacteur est le tréhalose. Le produit formé après hydrolyse enzymatique par les acyltransférases, en particulier par l'Ag85, est le p-aminophényl-β-D-glucopyranoside.

Selon un mode de réalisation particulier du procédé de détection de la présente invention, l'étape de détection électrochimique c) est réalisée à l'aide d'un capteur ampérométrique, modifié chimiquement (*e.g.* avec des nanotubes de carbone, du graphène) ou non. De préférence ledit capteur est un capteur sérigraphié, préférentiellement à usage unique.

Conformément à l'invention, un moyen d'analyse électrochimique pour la mise en œuvre de l'invention peut être une mesure potentiométrique, une mesure d'impédancemétrie, une mesure coulométrique ou une mesure ampérométrique.

Selon un mode de réalisation avantageux du procédé de détection de la présente invention, l'analyse électrochimique est mise en œuvre par une mesure ampérométrique.

On entend par « mesure ampérométrique » au sens de la présente invention, une mesure du courant électrique en fonction d'une différence de potentiel établie entre l'électrode de travail et l'électrode de référence.

La mesure du courant électrique peut être réalisée au moyen des techniques ampérométriques connues, préférentiellement par une voltampérométrie à balayage de potentiel pouvant être linéaire, cyclique, à impulsion, ou encore du type à saut de potentiel, tel que la chronoampérométrie.

Dans un mode de réalisation particulièrement avantageux du procédé de détection de la présente invention, la présence du p-aminophényl-β-D-glucopyranoside est mesurée par la voltampérométrie cyclique ou linéaire.

La mise en œuvre de ces techniques requiert un montage pouvant être à deux voire à trois électrodes, c'est-à-dire un montage comprenant une électrode de travail, une électrode de référence et éventuellement une électrode auxiliaire (contre-électrode). L'électrode de travail, dont la surface sert de site pour le transfert d'électrons, peut être à base de carbone ou à base d'un métal noble ou encore à base d'oxyde métallique. L'électrode de référence est une électrode dont le potentiel est constant, ce qui permet d'imposer un potentiel précisément défini à l'électrode de travail. L'électrode de référence peut être une électrode Ag/AgCl. La contre-électrode, qui permet d'établir le passage du courant électrique avec l'électrode de travail peut être fabriquée avec un matériau inerte, tel que le platine ou le carbone. L'homme du métier saura choisir et combiner les électrodes appropriées selon ses connaissances générales.

Concernant le mode de fabrication des électrodes, la technique de sérigraphie est préférable, bien que d'autres méthodes de fabrication industrielle telles que la rotagravure, l'impression à jet d'encre, 3D ou éventuellement la photolithographie puissent être envisagées. Les électrodes obtenues par sérigraphie sont particulièrement bien adaptées car elles peuvent être produites en masse à faible coût, et donc être éventuellement à usage unique. De plus, leur forme géométrique ainsi que leur taille peuvent être aisément modulables. Ces électrodes peuvent être sérigraphiées sous forme d'un capteur et éventuellement intégrées dans le fond des puits d'une microplaque ou d'autres supports ou systèmes permettant la filtration des suspensions bactériennes et l'incubation du p-aminophényl-6-O-octanoyl-β-D-glucopyranoside et du tréhalose.

Selon un mode de réalisation particulier du procédé de détection de la présente invention, la mesure ampérométrique est mise en œuvre avec un capteur sérigraphié. Il permet d'effectuer la mesure dans un petit volume de solution de l'ordre de quelques microlitres.

Selon un mode de réalisation particulier du procédé de détection de la présente invention, la mesure ampérométrique est mise en œuvre avec un dispositif impliquant trois électrodes : une électrode de référence Ag/AgCl, une électrode de travail en carbone et une contre-électrode en carbone.

Selon un autre mode de réalisation particulier du procédé de détection de la présente invention, la mesure ampérométrique est mise en œuvre avec un capteur sérigraphié comprenant une électrode de référence Ag/AgCl, une électrode de travail en carbone et une contre-électrode en carbone.

La présence du p-aminophényl-β-D-glucopyranoside est indiquée par la présence d'un courant d'oxydation anodique dans un intervalle de potentiels et l'absence dudit courant pour un contrôle dépourvu de p-aminophényl-6-O-octanoyl-β-D-glucopyranoside hydrolysé.

Lorsque le p-aminophényl-β-D-glucopyranoside est soumis à une mesure par voltampérométrie cyclique, sa présence est indiquée par un pic de courant d'oxydation anodique spécifique au p-aminophényl-β-D-glucopyranoside dans un intervalle de potentiels déterminé (+ 0,3 à + 0,5 V *vs.* Ag/AgCl).

De préférence, l'échantillon biologique à tester est préparé de sorte à isoler les mycobactéries qu'il contient tout en éliminant un maximum de contaminants avant les étapes de mise en contact et de détection. Pour ce faire, une étape d'extraction avec un solvant apolaire comme par exemple l'hexane est nécessaire pour isoler sélectivement les mycobactéries - dont la paroi est très hydrophobe - de l'échantillon préalablement mis en solution dans un fluidifiant comme la N-acétyl Cystéine pour un prélèvement respiratoire ou dans un tampon phosphate de pH neutre pour un échantillon de sol, par exemple. Dans le cas d'échantillons complexes comme le sol où peuvent coexister des millions d'espèces bactériennes différentes, l'étape d'extraction peut être suivie d'une décontamination de l'extrait avec par exemple un acide (HCl, $H_2SO_4$) et/ou une base (NaOH) ou un ammonium quaternaire.

Ainsi, la présente invention a également pour objet, un procédé d'isolement des mycobactéries d'un échantillon biologique, ledit procédé comprenant les étapes de :

a) mise en solution dudit échantillon biologique ;
b) traitement avec un solvant apolaire de la solution obtenue à l'étape a) ; et
c) récupération des mycobactéries par filtration ou centrifugation de la solution issue de l'étape b) ; et
d) récupération des mycobactéries à partir du filtrat ou du culot de centrifugation obtenu à l'issue de l'étape c).

Selon un mode de réalisation particulier du procédé d'isolement des mycobactéries de la présente invention, l'étape d'extraction b) est réalisée avec une solution d'hexane ou un mélange hexane-isopropanol.

Selon un mode de réalisation particulier du procédé d'isolement des mycobactéries de la présente invention, le procédé peut comprendre en outre une étape de décontamination a') de l'échantillon biologique mis en solution à l'issue de l'étape a) et avant l'étape b), et/ou une étape de décontamination c') de la membrane filtrante à l'issue de l'étape c) et avant l'étape d), avec des solutions acides (*e.g.* solution d'acide chlorhydrique) et/ou basiques (*e.g.* solution de soude ou d'ammonium quaternaire), et/ou ajout d'hypochlorite de sodium, et/ou avec au moins un autre composé désinfectant (*e.g.* chlorhexidine ou squalamine).

Selon un mode de réalisation particulier du procédé d'isolement des mycobactéries de la présente invention, l'étape c') peut être suivie avant l'étape d) d'une étape de rinçage c"), par exemple en présence de tampon phosphate, afin d'éliminer l'hypochlorite de sodium (eau de javel) du filtre (de préférence en téflon).

Selon un mode de réalisation particulier du procédé d'isolement des mycobactéries de la présente invention, l'étape d) de récupération des mycobactéries est réalisée par grattage du filtre avec une oese (anse) pour décoller les cellules du filtre. Les mycobactéries ainsi récupérées sont ensuite mises en culture dans un milieu approprié (milieu 7H11 enrichi et supplémenté), pendant environ deux mois, à 37°C, afin de permettre leur dénombrement.

La présente invention a également pour objet un kit pour la mise en œuvre du procédé de détection électrochimique des mycobactéries dans un échantillon biologique selon la présente invention, ledit kit comprenant :

a) un dispositif et les réactifs pour la collecte et la préparation de l'échantillon biologique à tester ;

b) un dispositif comprenant un substrat de l'Ag85 et son cofacteur pour l'incubation avec l'Ag85 ;

c) un dispositif pour la détection électrochimique à l'aide d'un lecteur adapté.

Par « dispositif » de l'étape a) au sens de la présente invention, on entend un contenant étanche, de préférence jetable, par exemple un tube ou une colonne à usage unique équipé d'un système de filtration dans lequel sont mises en œuvre les étapes de dilution de l'échantillon, d'extraction, de décontamination si nécessaire et de récupération des mycobactéries.

Par « dispositif » de l'étape b) au sens de la présente invention, on entend un contenant étanche, de préférence à usage unique, par exemple un tube à usage unique équipé d'un système de filtration dans lequel est mise en œuvre l'incubation des mycobactéries avec le substrat et le co-substrat.

Par « dispositif pour la détection électrochimique » de l'étape c) au sens de la présente invention, on entend par exemple un capteur ampérométrique, de préférence sérigraphié et à usage unique, par exemple ceux commercialisés par les sociétés Dropsens et Palmsens. Le capteur ampérométrique peut éventuellement être intégré dans le dispositif de l'étape b. A titre d'exemple de lecteur adapté, on peut citer des lecteurs portables basés sur le principe du lecteur de glycémie, par exemple ceux commercialisés par les sociétés Dropsens et Palmsens et qui permettent de réaliser les mesures en quelques secondes.

**Brève description des figures**

La figure 1 représente le schéma de principe de la réaction enzymatique catalysée par l'Ag85 avec le p-AP-OG et le tréhalose comme substrat et cosubstrat, respectivement.

La figure 2 représente les voltammogrammes linéaires (v = 50 mV.s$^{-1}$) d'une solution de substrat (p-AP-OG ; S) et de produit (p-AP-G ; courbe en pointillés ; P) à 5 × 10$^{-4}$ M dans du PBS (pH 7,5) - DMSO 0,2 %.

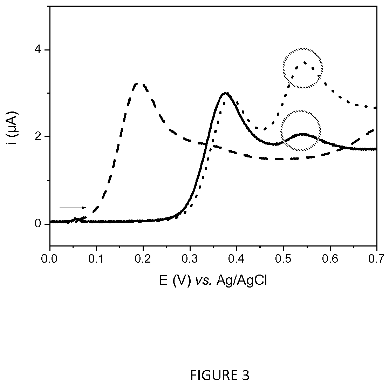

La figure 3 représente les voltammogrammes linéaires (v = 50 mV.s$^{-1}$) enregistrés pour une solution d'Ag85 (16 μg.mL$^{-1}$) incubée pendant 4h à 37°C avec le p-AP-OG (2 × 10$^{-4}$ M) et le tréhalose (10 mM) dans PBS en présence (courbe pointillée) et en absence (courbe en trait continu) de p-AP-G (10$^{-4}$ M). La courbe en trait - - - correspond au voltammogramme obtenu après incubation dans les mêmes conditions d'une solution de p-AP-OG (2 × 10$^{-4}$ M) dans PBS. Volumes réactionnels = 15 μL.

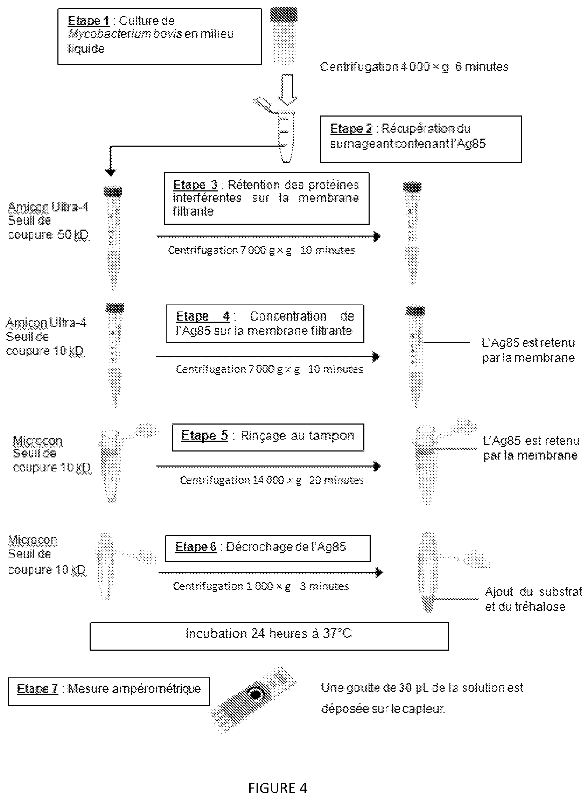

La figure 4 représente les étapes mises en œuvre lors de la détection de l'Ag85 dans le surnageant d'une culture de mycobactéries.

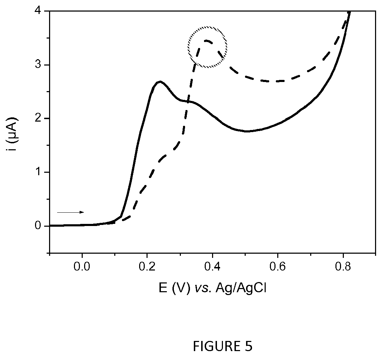

La figure 5 représente les voltammogrammes linéaires (v = 50 mV.s$^{-1}$) enregistrés après la mise en œuvre du protocole de la Figure 4 pour l'analyse de 5 mL de milieu 7H9 (courbe en trait continu) et 5 mL de culture de *M. bovis* BCG (1,1 × 10$^7$ ufc.mL$^{-1}$).

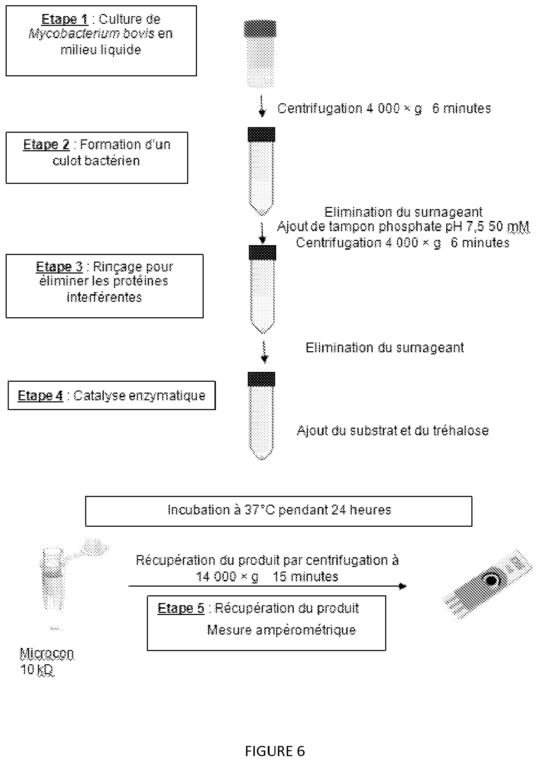

La figure 6 représente les étapes mises en œuvre lors de la détection de l'Ag85 dans le culot bactérien résultant d'une culture de mycobactéries.

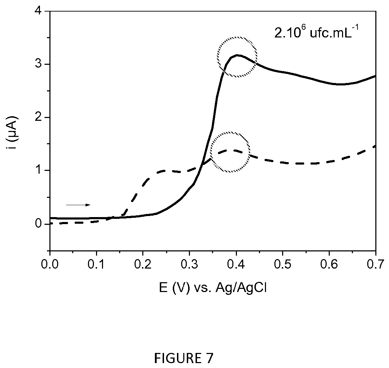

La figure 7 représente les voltammogrammes linéaires (v = 50 mV.s$^{-1}$) enregistrés en analysant des volumes 10 mL d'une culture liquide de *M. bovis* BCG à 2 × 10$^6$ ufc.mL$^{-1}$ selon les protocoles des Figures 4 et 6. La courbe en trait - - - correspond à la réponse enregistrée pour le surnageant et celle en trait continu a été obtenue pour l'analyse des culots bactériens.

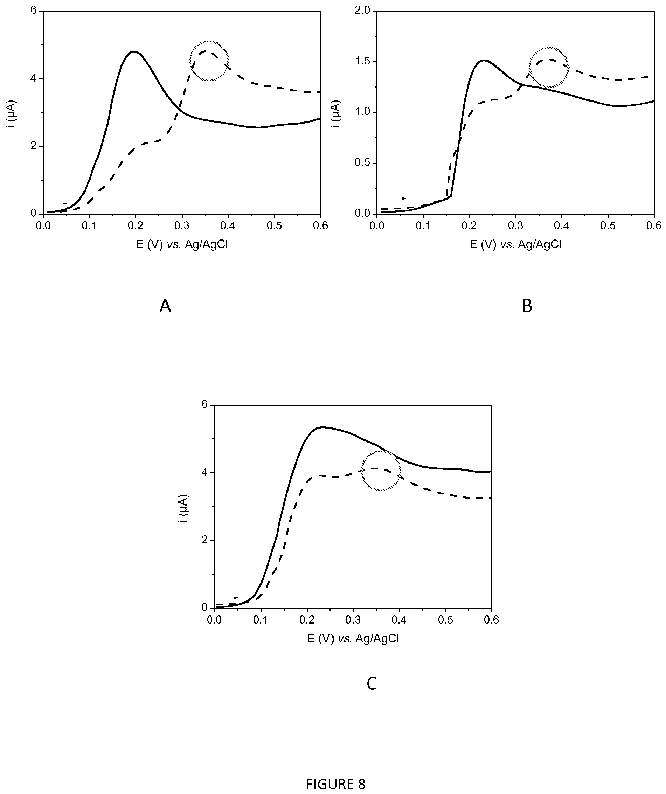

La figure 8 représente les voltammogrammes linéaires (v = 50 mV.s$^{-1}$) enregistrés en analysant 1 mL de milieu de culture 7H9 + OADC (contrôle négatif ; courbe en trait continu) contenant (A) *M. intracellulare,* (B) *M. avium* et (C) *M. xenopi* à ~ 10$^6$ bacilles.mL$^{-1}$ (courbes en trait - - -) analysés selon le protocole décrit dans le paragraphe 1.5.

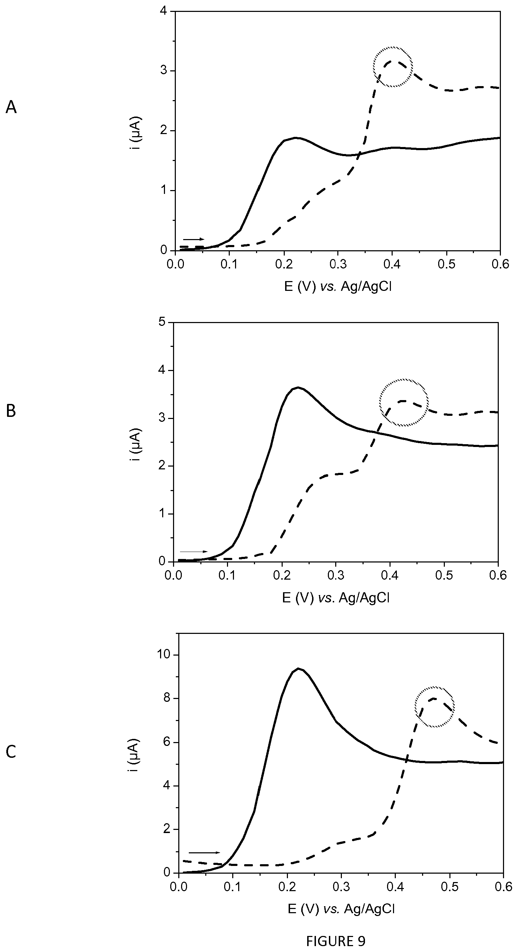

La figure 9 représente les voltammogrammes linéaires (v = 50 mV.s$^{-1}$) enregistrés pour l'analyse qualitative d'une culture liquide de *M. tuberculosis* (A) surnageant de culture, (B) culot bactérien et celle (C) de colonies isolées de *M. tuberculosis* selon le protocole décrit dans le paragraphe 1.6. Les courbes en trait - - - correspondent à la réponse *M. tuberculosis* tandis que les courbes en trait continu ont été obtenues pour le contrôle négatif.

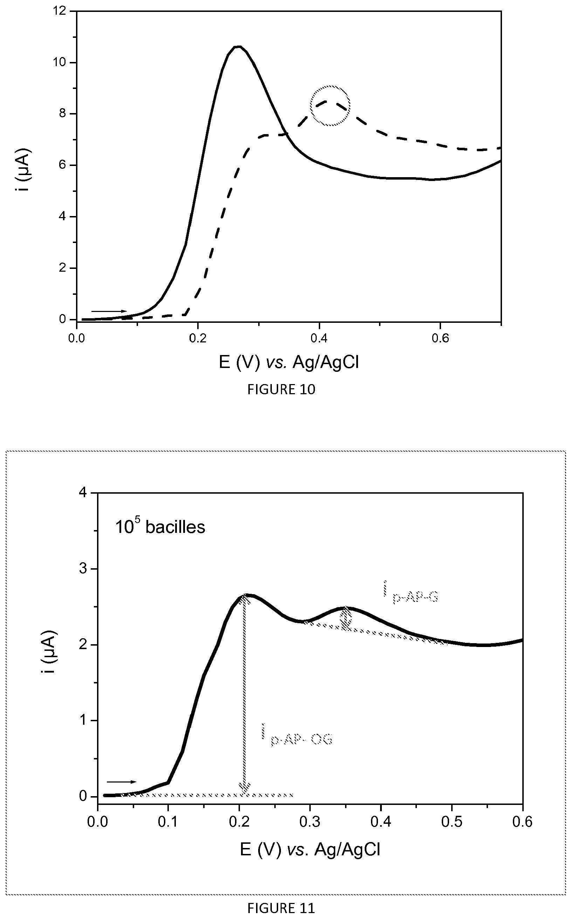

La figure 10 représente les voltammogrammes linéaires (v = 50 mV.s$^{-1}$) enregistrés après la mise en œuvre du protocole décrit dans le paragraphe 1.7 pour l'analyse d'1 mL de crachat inoculé avec 10$^6$ bacilles de *M. intracellulare* (courbe en pointillés) ou non inoculé (courbe en trait continu).

La figure 11 représente le voltammogramme linéaire (v = 50 mV.s$^{-1}$) enregistré en analysant 1 mL de culture de *M. intracellulare* à ~ 10$^5$ bacilles.mL$^{-1}$ selon le protocole décrit dans le paragraphe 1.5. ainsi que les valeurs i p-AP-OG et i p-AP-G nécessaires au calcul de la réponse analytique R.

**EXEMPLES**

**Exemple 1 : Matériel et Méthodes**

**1.1. Réactifs et solutions**

- Le *p*-aminophényl-6-O-octanoyl-β-D-glucopyranoside ($C_{20}H_{31}NO_7$; p-AP-OG) ainsi que le *p*-aminophényl-6-β-D-glucopyranoside ($C_{12}H_{17}NO_6$ ; p-AP-G) ont été synthétisés à l'Institut de Chimie Moléculaire de l'Université de Bourgogne.
- Le tréhalose et le diméthylsulfoxyde (DMSO) ont été fournis par Sigma-Aldrich.
- L'Ag85B de *Mycobacterium tuberculosis* (Ag 85 ; ab73632) a été acheté chez Abcam et reconstitué selon les recommandations du fournisseur.
- L'hexane et l'isopropanol proviennent du laboratoire Carl Roth.
- Le tampon phosphate (16,7 mM $NaH_2PO_4$, 2 $H_2O$ ; 33,3 mM $Na_2HPO_4$, 12 $H_2O$ - pH 7,5 50 mM) a été préparé avec de l'eau Milli-Q 18 MΩ (Système Millipore).
- Les milieux liquide (Middlebrook 7H9 Broth Base, Fluka) et solide (Middlebrook 7H11 Agar Base, Fluka), ainsi que les constituants du produit d'enrichissement (acide oléïque, albumine bovine, dextrose et catalase) ont été fournis par Sigma-Aldrich. Le tryptone (peptone de caséine) a été fourni par VWR. Le sérum bovin inactivé à la chaleur a été fourni par Dutscher.
- Les tubes de milieu liquide BD BACTEC™ MGIT™ (mycobacterial growth indicator tubes) ont été fournis par Becton Dickinson, ainsi que le supplément de croissance BACTEC™ MGIT™ et le complexe d'antibiotiques BBL MGIT™ PANTA lyophilisé. Le milieu a été préparé selon les recommandations du distributeur.

**1.2. Souches et milieux de culture**

- Les souches de *Mycobacterium intracellulare, M. avium ssp. avium, M. bovis* BCG souche Pasteur (souche vaccinale avirulente) et *M. xenopi* ont été fournies par le Laboratoire National de Référence pour la tuberculose bovine de Maisons-Alfort. La souche *M. avium ssp. paratuberculosis* K10 a été fournies par l'INRA de Tours. Ces souches ont été manipulées dans un laboratoire de confinement de type L2.
- La souche de *M. tuberculosis* H37Rv provient du Laboratoire associé au Centre National de Référence des Mycobactéries et de la Résistance des Mycobactéries aux Antituberculeux (Hôpitaux Universitaires St Louis-Lariboisière-F. Widal). Comme les souches de *M. tuberculosis* appartiennent à la catégorie de risques infectieux « Classe 3 », toutes les expériences avec la souche *M. tuberculosis* H37Rv ont été réalisées dans un laboratoire de type L3.
- Les souches de *Staphylococcus aureus* (DMSZ20231), *Staphylococcus epidermidis* (DSMZ20044), *Pseudomonas monteilii* (DMSZ14164), Enterococcus faecalis (DMSZ20478), *Enterococcus faecium* (DMSZ20477), *Escherichia coli* (DMSZ30083), et *Stenotrophomonas maltophilia* (DMSZ50170) ont été commandées au Leibniz Institute DSMZ-German Collection of Microorganisms and Cell Cultures.
- Les souches de *Streptococcus pyogenes, Pseudomonas aeruginosa, Pseudomonas fluorescens, Rhodococcus corallinus, Achromobacter xylosoxidans, Citrobacter freundii, Enterobacter cloacae* et *Klebsiella pneumoniae* ont été gracieusement fournies par l'INRA et le CHU de Dijon.
- Le milieu de culture liquide a été préparé en diluant 5,9 g de Middlebrook 7H9 Broth Base et 1,25 g de tryptone dans 1 L d'eau milli-Q puis autoclavé 15 minutes à 121 degrés.
- Le milieu de culture solide a été préparé en diluant 18,9 g de Middlebrook 7H11 Agar Base dans 800 mL d'eau milli-Q puis autoclavé 15 minutes à 121 degrés.

Dans le but de favoriser la croissance des mycobactéries, les milieux de culture liquide et solide ont été enrichis avec 10% d'un mélange constitué d'acide oléïque, d'albumine, de dextrose et de catalase (OADC ; Tableau 1). Le milieu 7H11 est également supplémenté avec 10% de sérum bovin inactivé à la chaleur. L'acide oléïque et les acides gras à longue chaîne sont essentiels pour le métabolisme des mycobactéries. Le dextrose est une source énergétique. La catalase permet une neutralisation des peroxydes, qui peuvent être toxiques pour les bactéries. L'albumine joue un rôle de protection contre les agents toxiques.

**Tableau 1.** Composition de l'enrichissement OADC

| Composants | |
|---|---|
| Dextrose | 20,0 g |
| Albumine bovine | 50,0 g |
| Acide oléique* | 0,6 |
| Catalase* | 0,003 g |
| Eau | 1 L |

**1.3. Capteurs et appareil de mesure**

Les mesures électrochimiques ont été réalisées par voltammétrie linéaire (v = 50 mV.s$^{-1}$) avec un potentiostat 910 PSTAT mini (Metrohm, France) alimenté par la connection USB de l'ordinateur et piloté par le logiciel PSTAT (version 1.0). Pour ce faire, des gouttes de solution de 30-50 $\mu$L ont été déposées sur la surface de capteurs sérigraphiés en carbone à usage unique fournis par Dropsens (DRP-110) et reliés au potentiostat *via* le connecteur (DRP-DSC). La détection ampérométrique du produit généré au cours de la réaction catalysée par l'Ag85 a toujours été réalisée après une étape de filtration du mélange réactionnel avec un dispositif de filtration (Microcon 10kDa, Millipore). Tous les potentiels sont mesurés par rapport à l'électrode de référence Ag/AgCl.

**1.4. Détection de l'Ag85 dans le surnageant d'une culture liquide de *M. bovis* BCG**

Le principe de ce protocole en 7 étapes est schématisé Figure 4. Quatre mL d'une culture de *M. bovis* BCG âgée de trois semaines ont été centrifugés à 4 000 $\times$ g pendant 6 min (étape 1). Le surnageant a été récupéré (étape 2) et déposé dans le réservoir d'un dispositif de filtration Amicon® Ultra 4 mL (porosité de 50 kD laissant passer le Complexe Ag85, Merck Millipore, France) afin d'éliminer les protéines interférentes du milieu de culture, notamment la BSA et la catalase, et centrifugé 10 min. à 7 000 $\times$ g (étape 3). Le filtrat est déposé dans le réservoir d'un second Amicon® Ultra 4 mL de porosité 10 kD permettant la concentration du Complexe Ag85 après centrifugation 10 min. à 7 000 $\times$ g. La membrane filtrante est rincée avec 500 $\mu$L de PBS et centrifugée selon les mêmes paramètres que précédemment (étape 4). Le volume retenu par la membrane (200-250 $\mu$L) est ensuite déposé dans un dispositif Microcon® de porosité 10 kD, centrifugé 20 min. à 14 000 $\times$ g puis rincé avec PBS (étape 5). Le dispositif de filtration est retourné et centrifugé 3 min. à 1 000 $\times$ g afin de décrocher le Complexe Ag85 (volume final d'environ 80 $\mu$L) (étape 6). 10 $\mu$L d'une solution de tréhalose à 5 $\times$ 10$^{-3}$ M dans PBS et 10 $\mu$L d'une solution de p-AP-OG à 2 $\times$ 10$^{-3}$ M dans PBS sont finalement ajoutés au filtrat. Après une incubation à 37°C pendant 24 h, le milieu réactionnel est filtré sur un dispositif Microcon® de porosité 10 kD (15 min. à 14 000 $\times$ g) puis 30 $\mu$L de filtrat sont déposés sur le capteur et analysés par voltammétrie (étape 7).

**1.5. Détection de l'Ag85 dans le culot bactérien obtenu à partir des cultures liquides de *M. intracellulare, M. avium* et *M. xenopi***

Des volumes de 1 mL de culture liquide de chaque espèce de mycobactérie ($\sim$ 10$^6$ bacilles.mL$^{-1}$) ont été centrifugés à 6 000 x g pendant 6 minutes. Une fois le surnageant éliminé, les culots bactériens ont été rincés avec 1 mL de PBS puis centrifugés à 6 000 x g pendant 6 minutes. Après élimination du surnageant, 10 $\mu$L de p-AP-OG à 2 $\times$ 10$^{-3}$ M, et 10 $\mu$L de tréhalose à 5 $\times$ 10$^{-3}$ M sont ajoutés aux culots bactériens. Après une étape d'incubation de 4 heures à 37°C avec agitation, la mesure électrochimique du produit de la réaction enzymatique a été réalisée selon le protocole décrit dans le paragraphe 1.3.

Un contrôle négatif (milieu 7H9 enrichi sans bactéries) a été analysé en double de la même manière.

**1.6. Détection de l'Ag85 chez *M. tuberculosis***

1.6.1. La culture liquide

Deux échantillons de 7,5 mL de culture liquide de *M. tuberculosis* ($\sim$ 10$^7$ bacilles.mL$^{-1}$) ont été centrifugés à 7 000 x g pendant 10 minutes. Afin d'analyser séparément le culot bactérien et le surnageant, ce dernier a été transféré dans des tubes stériles.

*Analyse du culot bactérien* :

Chaque culot bactérien a été rincé en ajoutant 100 µL de PBS puis le liquide a été éliminé par retournement du tube sur un papier absorbant (Whatman). 20 µL de p-AP-OG à $2 \times 10^{-3}$ M et 20 µL de tréhalose à $5 \times 10^{-3}$ M ont été ajoutés dans les tubes et incubés 4 heures à 37°C sans agitation. Des témoins négatifs (PBS) ont été analysés en double de la même manière. La mesure électrochimique du produit de la réaction enzymatique a été réalisée selon le protocole décrit dans le paragraphe 1.3.

*Analyse du surnageant :*

Six mL de chaque surnageant de culture ont été centrifugés à 7 000 x g pendant 20 min dans un dispositif de filtration Amicon 50 kDa (Millipore). Le liquide filtré a été transféré dans un dispositif de filtration Amicon 10 kDa puis centrifugé à 7 000 x g pendant 20 min. Le filtre a alors été rincé avec 1 mL de PBS puis centrifugé à 7 000 x g pendant 20 min. Le volume de liquide résiduel restant sur le filtre a été transféré dans un tube dans lequel ont été ajoutés 30 µL de p-AP-OG à $2 \times 10^{-3}$ M et 30 µL de tréhalose à $5 \times 10^{-3}$ M. Le mélange réactionnel a été incubé 4 heures à 37°C sans agitation. Un contrôle négatif (milieu 7H9 enrichi sans bactéries) a été analysé en double de la même manière. La mesure électrochimique du produit de la réaction enzymatique a été réalisée selon le protocole décrit dans le paragraphe 1.3.

1.6.2. Les colonies isolées

Quelques colonies prélevées sur une gélose 7H11 ont été déposées dans un tube de 2 mL. Les colonies ont été rincées au PBS, les tubes centrifugés et le surnageant éliminé. 20 µL de p-AP-OG à $2 \times 10^{-3}$ M et 20 µL de tréhalose à $5 \times 10^{-3}$ M ont été ajoutés dans les tubes et incubés 4 heures à 37°C sans agitation. Un témoin négatif (PBS) a été analysé en double de la même manière. La mesure électrochimique du produit de la réaction enzymatique a été réalisée selon le protocole décrit dans le paragraphe 1.3.

**1.7. Extraction et détection de *M. intracellulare* dans un échantillon d'origine respiratoire**

Des prélèvements respiratoires (crachats, produits d'aspiration trachéale et d'aspiration bronchique) de patients non tuberculeux ont été fournis par le CHU de Dijon. Les échantillons ont été préalablement fluidifiés par le CHU de Dijon avec le kit Digest-EUR (Eurobio).
Des volumes de 1 mL d'échantillon respiratoire ont été distribués dans des tubes stériles de 15 mL puis incubés avec 200 µL d'une culture liquide de *M. intracellulare* contenant $10^6$ bacilles ou 200 µL de milieu liquide stérile (contrôle négatif) pendant 1 nuit à 37°C.

*Extraction de M. intracellulare*

9 mL de mélange hexane-isopropanol (3:2, v/v) ont été ajoutés dans chaque tube de prélèvement respiratoire et agités pendant 1 minute. Après une étape de centrifugation à 3 000 x g pendant 2 minutes, le surnageant (c'est-à-dire l'hexane, et l'interface) a été prélevé puis filtré sous vide sur une membrane (Durapore, 25 mm ; 0,45 µM). Une fois rincée au PBS, la membrane a été déposée dans un petit sachet en polyéthylène avec fermeture ZIP soudé aux dimensions de la membrane.

*Détection électrochimique de M. intracellulare dans l'extrait*

Un volume de 100 µL du mélange substrat à $2 \times 10^{-3}$ M et tréhalose à $5 \times 10^{-3}$ M préparé dans PBS a été introduit dans le sachet avant sa fermeture. Après une étape d'incubation à 37°C pendant 4 heures, la mesure électrochimique du produit de la réaction enzymatique a été réalisée selon le protocole décrit dans le paragraphe 1.3.

**1.8. Extraction et détection de *M. bovis* BCG dans un échantillon de sol**

*Extraction de M. bovis BCG*

Les microcosmes de sol ont été préparés à partir d'un sol argilo-limoneux de pH 7,75 autoclavé deux fois 15 min à 121°C à 48 h d'intervalle pour s'affranchir de la flore microbienne endogène. Des microcosmes de 5 g de sol stérile ont été préparés dans des tubes Falcon de 45 mL, puis inoculés avec *M. bovis* BCG ($2,6 \times 10^6$ ufc). Après 12 h d'incubation à température ambiante dans l'obscurité, chaque microcosme a été soumis au protocole d'extraction suivant.

Un volume de 15 mL de tampon phosphate 0,1 M a été ajouté à chaque microcosme puis le sol a été remis en suspension par une agitation de 2 min. au vortex. Ensuite, un volume de 10 mL d'hexane a été ajouté à la suspension de sol pour extraire sélectivement les microorganismes du sol possédant une enveloppe hydrophobe. Ce mélange a été agité pendant 15 min sur un agitateur rotatif. Chaque tube a été centrifugé pendant 10 min à 4 000 × g (swinging bucket rotor, centifugeuse Beckman GS-15R) afin de séparer les différentes phases liquides et de sédimenter les particules de sol au fond du tube. L'interface contenant les microorganismes ciblés est située entre la phase aqueuse et la phase organique. Elle a été prélevée avec une pipette et un cône d'1 mL dont la pointe a été coupée puis déposée sur une membrane filtrante de 2,5 cm en téflon de porosité 0,45 μM (Durapore, Millipore, France). La membrane a été soumise à une filtration par aspiration sous vide afin d'éliminer la phase liquide et de concentrer les bactéries sur la membrane. Celle-ci a été placée dans un pilulier contenant 1 mL de tampon phosphate pour l'étape de décontamination.

*Décontamination de l'extrait et dénombrement*

Cette étape est très utile voire indispensable pour éliminer la flore microbienne édaphique interférente du sol. Pour ce faire, le protocole de décontamination a associé une décontamination acide (ajout de 100 μL d'acide chlorhydrique 4 %, incubation pendant 20 min.) avec un pH estimé de 1,3 et une décontamination basique (ajout de 200 μL de soude 4 %, incubation pendant 20 min.) avec un pH estimé de 12,6, puis le mélange a été neutralisé par l'ajout de 100 μL d'acide chlorhydrique avant l'ajout de 1 mL de mélange d'hypochlorite de sodium et de soude. Après 15 minutes d'incubation, le surnageant a été prélevé, déposé sur une nouvelle membrane filtrante en téflon et rincé avec 20 mL de tampon phosphate 0,1 M pour éliminer la javel. La membrane a alors été déposée dans un nouveau pilulier contenant 1 mL de 7H9 enrichi et grattée avec une oese (anse) pour décoller les cellules du filtre. En vue de dénombrer les mycobactéries extraites, 100 μL de chaque suspension finale ont été ensemencés en triplicat sur 7H11 enrichi et supplémenté, et incubés deux mois à 37°C.

**Exemple 2 : Résultats**

**2.1. Comportement voltammétrique du p-AP-OG et du p-AP-G**

Les voltammogrammes présentés Figure 2 montrent qu'il est possible de distinguer la réponse voltammétrique du substrat p-AP-OG (S) de celle du produit p-AP-G (P). En effet, les pics d'oxydation détectés à des potentiels de ~ + 0,2 V et ~ + 0,4 V (*vs.* Ag/AgCl) ont été respectivement enregistrés pour le p-AP-OG et le p-AP-G. Cette différence de potentiel de pic, liée à la présence ou à l'absence du groupement électroattracteur octanoyl, indique que le p-AP-OG peut être utilisé comme substrat pour la détection ampérométrique de l'activité acyltransférase de l'Ag85.
En effet, comme le montre le schéma de la Figure 1, l'Ag85 est capable d'hydrolyser la liaison ester du p-AP-OG et de transférer le groupement octanoyl sur le tréhalose pour respectivement générer le p-AP-G et l'acyl-tréhalose selon un mécanisme ping-pong. Ainsi, l'intensité du pic d'oxydation du p-AP-G mesurée vers ~ + 0,4 V vs. Ag/AgCl peut être choisie comme réponse analytique puisque sa valeur est proportionnelle à la quantité de p-AP-G produit, donc à celle d'Ag85 et indirectement à celle des mycobactéries présentes dans l'échantillon à analyser.

**2.2. Détection électrochimique de l'activité acyltransférase de la protéine Ag85**

Afin de démontrer l'activité acyltransférase de l'Ag85 en présence de p-AP-OG et d'un accepteur de groupements acyles, le mélange réactionnel contenant la protéine Ag85, le p-AP-OG et le tréhalose a été incubé en présence et en absence de p-AP-G sous agitation pendant 4 heures à 37°C. Parallèlement, un contrôle négatif contenant uniquement le p-AP-OG a été analysé dans les mêmes conditions.
Comme le montre la série de voltammogrammes présentée Figure 3, la courbe enregistrée pour le contrôle négatif (courbe en trait - - -) présente un potentiel de pic d'oxydation du p-AP-OG vers ~ + 0,2 V *vs.* Ag/AgCl alors que la réponse voltammétrique obtenue en présence de l'Ag85 (courbe en trait continu) possède deux pics d'oxydation situés vers + ~ 0,4 et 0,55 V *vs.* Ag/AgCl, respectivement.
Une étude complémentaire (résultats non présentés) a montré que la valeur du potentiel de pic d'oxydation du p-AP-OG augmente en fonction de la concentration en ions chlorures dans la solution. Par ailleurs, les informations concernant l'Ag85 commercialisé indiquent que la protéine a été lyophilisée à partir d'un tampon contenant 0,1 M NaCl. Ainsi, les valeurs plus élevées des potentiels de pic d'oxydation du p-AP-OG et du p-AP-G enregistrées en présence d'Ag85 sont probablement dues à la celle des chlorures dans la solution.
Afin de valider cette hypothèse, l'Ag85 a également été incubé en présence de tréhalose et d'un mélange de p-AP-OG et p-AP-G. La comparaison des voltammogrammes (courbe en trait continu et courbe en pointillés) de la Figure 3 confirme l'identité de chaque pic et montre qu'il est possible d'envisager la détection électrochimique de l'Ag85 à l'aide du p-AP-OG comme substrat.

**2.3. Détection électrochimique de l'activité acyltransférase de l'Ag85 dans une culture liquide de *M. bovis* BCG**

Dans le but d'appliquer la méthode de détection électrochimique de l'Ag85 pour mettre en évidence la croissance des mycobactéries dans un milieu de culture liquide, l'analyse du surnageant d'une culture de *M. bovis* BCG strain Pasteur (souche vaccinale et modèle avirulente des mycobactéries tuberculeuses) ainsi que celle du culot bactérien ont été envisagées. Les concentrations en substrat et en co-substrat, la durée de la réaction de catalyse enzymatique ainsi que la nécessité d'inclure une étape de filtration avant la mesure électrochimique ont été étudiées dans une série d'expériences préliminaires.

2.3.1. Analyse du surnageant

1. Il a été démontré que l'Ag85 est un produit de sécrétion majeur de *M. tuberculosis* en phase réplicative (Wiker et Harboe, Microbiol. Rev., 56(4) : 648-661, 1992) [9]. Le protocole schématisé Figure 4 et décrit dans le paragraphe 1.4 a été mis en œuvre pour analyser 5 mL d'une culture de *M. bovis* BCG contenant $1,1 \times 10^7$ ufc.mL$^{-1}$. Un contrôle négatif (milieu 7H9 enrichi sans bactéries) a été analysé en parallèle selon le même protocole et les résultats sont présentés Figure 5. La réponse voltammétrique du contrôle négatif (courbe en trait continu) correspond globalement au pic d'oxydation du p-AP-OG situé vers ~ + 0,25 V *vs.* Ag/AgCl tandis que celle enregistrée pour le surnageant de culture (courbe en trait - - -) présente non seulement le pic d'oxydation du p-AP-OG (avec une intensité plus faible) mais également le pic d'oxydation du p-AP-G. La présence de ce dernier indique la présence d'Ag85 dans le surnageant de culture et donc celle des mycobactéries dans la culture analysée.

2.3.2. Analyse du culot bactérien

L'Ag85 intervenant également dans la réparation et la construction de la paroi des mycobactéries, le protocole schématisé Figure 6 a été mis en œuvre pour analyser 10 mL d'une culture de *M. bovis* BCG contenant $2 \times 10^6$ ufc.mL$^{-1}$. Parallèlement, le surnageant de cette même culture a été analysé comme précédemment.

Le voltammogramme (courbe en trait - - -) présenté Figure 7 confirme la détection de l'activité de l'Ag85 dans le surnageant de culture avec la présence des pics d'oxydation du p-AP-OG et du p-AP-G. D'autre part, la réponse voltammétrique enregistrée pour l'analyse du culot bactérien (courbe en trait continu) présente uniquement le pic d'oxydation du p-AP-G avec une forte intensité. Ce résultat indique que la totalité du substrat p-AP-OG a été converti en p-AP-G pendant l'étape d'incubation et suggère donc que le culot bactérien contient une quantité d'Ag85 plus importante que le surnageant pour un volume de culture donné. Une étude complémentaire sur la spécificité de la méthode a montré que les acyltransférases présentes chez *Pseudomonas aeruginosa*, *Pseudomonas fluorescens*, *Pseudomonas monteilii*, *Staphylococcus aureus*, *Staphylococcus epidermidis*, *Streptococcus pyogenes*, *Rhodococcus corallinus*, *Achromobacter xylosoxidans*, *Citrobacter freundii*, *Enterobacter cloacae*, *Enterococcus faecalis*, *Enterococcus faecium*, *Klebsiella pneumoniae* et *Stenotrophomonas maltophilia* sont capables de transformer le p-AP-OG en p-AP-G en présence de tréhalose.

Ainsi, la réponse ampérométrique du p-AP-G mesurée lors de l'analyse du culot bactérien de *M. bovis* BCG résulte probablement d'une part de l'activité acyltransférase de l'Ag85 présent dans l'enveloppe mycobactérienne et d'autre part des acyltransférases contenues dans les mycobactéries.

**2.4. Détection électrochimique de plusieurs espèces de mycobactéries dans des cultures liquides.**

La preuve de concept de la détection électrochimique des mycobactéries en présence de p-AP-OG et de tréhalose a été mise œuvre pour l'analyse de plusieurs espèces de mycobactéries manipulables dans un laboratoire de confinement L2. Les espèces sélectionnées sont celles fréquemment retrouvées dans des prélèvements respiratoires : *M. intracellulare, M. avium* ssp. avium et *M. xenopi*. Pour ce faire, le protocole décrit dans le paragraphe 1.4 a été mis en œuvre et les résultats obtenus pour l'analyse des culots bactériens contenant approximativement $10^6$ bacilles sont présentés dans la Figure 8. L'obtention d'un pic d'oxydation spécifique du produit d'hydrolyse du substrat par l'Ag85 (p-AP-G) pour l'analyse de chaque espèce (courbes en trait - - - , E - + 0,4 V *vs.* Ag/AgCl) confirme qu'il est possible de détecter, avec une méthode électrochimique, la présence de mycobactéries avec le p-AP-OG comme substrat et le tréhalose comme co-substrat. Pour chaque espèce, des seuils de quantification compris entre $10^3$ et $10^4$ bactéries.mL$^{-1}$ de culture ont été estimés.

**2.5. Détection de *M. tuberculosis***

La détection électrochimique de *M. tuberculosis,* microorganisme de classe 3 responsable de la tuberculose humaine, a été réalisée en analysant des cultures de la souche H37Rv en milieu liquide 7H9 et sur milieu gélosé 7H11 selon les modes opératoires du paragraphe 1.6. Dans le cas de la culture liquide, la détection de l'Ag85 a été envisagée dans le culot bactérien ainsi que dans le surnageant de culture.

Avec un pic d'oxydation spécifique du produit d'hydrolyse du p-AP-OG, la série de voltammogrammes présentés Figure 9 A confirme que la souche de *M. tuberculosis* produit de l'Ag85 en grande quantité dans le milieu de culture. Ces résultats confirment qu'il est possible d'envisager la détection de *M. tuberculosis via* la mesure électrochimique de l'activité de l'Ag85 dans le milieu de culture.

Par ailleurs, les pics d'oxydation du p-AP-G enregistrés pour l'analyse des cellules bactériennes (Figures 9B, 9C ; courbes en trait - - -) indiquent que la méthode électrochimique impliquant le p-AP-OG comme substrat et le tréhalose comme accepteur de groupements acyles permet également la détection de *M. tuberculosis* sous forme de culots bactériens et de colonies isolées.

## 2.6. Extraction et détection de *M. intracellulare* dans un échantillon respiratoire

Afin de proposer une méthode de détection électrochimique directe (c'est-à-dire sans culture préalable) des mycobactéries dans des échantillons d'origine pulmonaire (crachats, produits d'aspiration trachéale et d'aspiration bronchique), des prélèvements de patients non tuberculeux ont été inoculés avec une quantité connue de *M. intracellulare* et analysés selon le protocole du paragraphe 1.7.

Comme les méthodes de fluidification-décontamination commercialisées utiles à la préparation des échantillons - qui associent la N-acétylcystéine et la soude - ne sont pas compatibles avec la détection électrochimique (signaux mal définis), le développement d'un protocole d'extraction des mycobactéries dans les prélèvements d'origine pulmonaire a été envisagé. La méthode proposée implique un mélange hexane-isopropanol comme solvant d'extraction. En précipitant les constituants du prélèvement respiratoire, l'isopropanol permet de s'affranchir du caractère visqueux du prélèvement tandis que le solvant apolaire, qu'est l'hexane, extrait sélectivement les mycobactéries dont la paroi est très hydrophobe. Une fois récupérées par filtration, les mycobactéries ont été incubées avec le mélange substrat-cosubstrat en vue de réaliser la détection électrochimique de l'activité acyltransférase (Ag85 et autres enzymes présentes dans la cellule mycobactérienne).

Les voltammogrammes présentés Figure 10 montrent qu'il est possible de détecter les mycobactéries grâce à la méthode électrochimique dans des prélèvements d'origine respiratoire préalablement traités avec un mélange hexane-isopropanol. En effet, le pic d'oxydation spécifique du produit d'hydrolyse du p-AP-OG par l'Ag85 est obtenu pour le crachat inoculé avec *M. intracellulare* (courbe en trait - - -, E ~ +0,4 V *vs.* Ag/AgCl) tandis qu'aucun pic d'oxydation lié à la présence du p-AP-G n'a été enregistré pour le crachat non inoculé (courbe en trait continu).

## 2.7. Extraction et détection de *M. bovis* BCG dans un échantillon de sol

Afin d'évaluer l'impact du volume d'hexane sur le rendement d'extraction, du sol stérile (5 g) a été inoculé avec la souche *M. bovis* BCG ($1,5 \times 10^5$ ufc par microcosme) et soumis au protocole d'extraction décrit dans le paragraphe 1.8 en utilisant des volumes d'hexane de 2 mL, 5 mL et 10 mL. Le rendement maximal d'extraction a été obtenu en utilisant un volume d'hexane de 10 mL. Dans ces conditions, 50 % des mycobactéries inoculées dans les microcosmes ont été extraites. Ce bon rendement d'extraction est lié à l'affinité forte entre l'hexane et la membrane hydrophobe des mycobactéries. De plus, l'hexane a probablement un rôle dans la destruction des liaisons (physisorption, chimisorption) qui provoquent l'adhésion des mycobactéries avec les particules de sol.

Les caractéristiques particulières de *M. bovis,* et particulièrement sa croissance très lente, nécessitent généralement une étape de décontamination de l'extrait avant sa mise en culture pour éliminer la majorité des microorganismes endogènes des substrats environnementaux tout en préservant les mycobactéries. Plusieurs protocoles ont été évalués et seul celui combinant des variations de pH extrêmes et une incubation en présence d'hypochlorite de sodium a été suffisamment efficace pour détruire toute la flore microbienne interférente du sol co-extraite par l'hexane en même temps que *M. bovis* BCG. En appliquant le protocole d'extraction-décontamination décrit dans le paragraphe 1.8 à des échantillons de sol stérile inoculés (5 g) avec *M. bovis* BCG ($2,6 \times 10^6$ ufc par microcosme), un rendement de 2,5 $\pm$ 0,8 % (n = 4) a été obtenu. Ce résultat suggère que la décontamination est l'étape limitante dans la démarche puisqu'elle élimine 95 % des mycobactéries qui ont été extraites avec l'hexane. Cette étape est toutefois très utile voire indispensable pour s'affranchir des microorganismes contaminants possédant une enveloppe hydrophobe proche de celle de *M. bovis,* tels que les divers genres rencontrés chez les Actinobactéries*.*

Bien que la méthode d'extraction-décontamination des mycobactéries dans le sol de l'invention a permis de recouvrer seulement environ 2,5 % des bactéries inoculées dans du sol stérile, ce rendement est nettement supérieur à celui décrit dans la littérature pour l'analyse d'échantillons environnementaux naturellement contaminés avec un procédé d'immonucapture magnétique (0,1 %) (Sweeney et al., Lett. Appl Microbiol., 43(4) : 364-369, 2006; Sweeney et al., Appl Environ Microbiol., 73(22) : 7471-7473, 2007) [10, 11].

**2.8. Aspects quantitatifs de la méthode**

Afin de prendre en compte l'ensemble des erreurs aléatoires lors de la mise en œuvre des protocoles, le p-AP-OG a été choisi comme étalon interne et la réponse analytique de la méthode a été définie comme le rapport de l'intensité du pic d'oxydation du p-AP-G sur celle de l'intensité du pic d'oxydation du p-AP-OG :

$$R = \frac{i\,p{-}AP{-}G}{i\,p{-}AP{-}OG}$$

Les grandeurs $i_{p\text{-}AP\text{-}OG}$ et $i_{p\text{-}AP\text{-}G}$ ont été définies sur le voltammogramme présenté Figure 11.

Comme le montrent les valeurs de R rassemblées dans le Tableau 2 ci-dessous, la méthode électrochimique proposée par les inventeurs a permis de détecter des quantités de *M. intracellulare* inférieures à 100 bacilles.mL$^{-1}$. Ce résultat permet d'envisager des applications de la méthode pour l'analyse d'échantillons réels, après extraction de mycobactéries, sans avoir recours à une étape de culture au préalable.

**Tableau 2** : Réponses analytiques R normalisées (R$_0$ correspond à la réponse analytique enregistrée pour le milieu de culture sans bactérie) calculées à partir des voltammogrammes enregistrés pour l'analyse de *M. intracellulare* à $10^2$, $10^3$, $10^4$, $10^5$ et $10^6$ bacilles.mL$^{-1}$ selon protocole décrit dans le paragraphe 1.4.

| *M. intracellulare* (bacille.mL$^{-1}$) | R/R$_0$ |
|---|---|
| 0 | 1 |
| $10^2$ | 2,9 |
| $10^3$ | 4,5 |
| $10^4$ | 7,4 |
| $10^5$ | 12,2 |
| $10^6$ | 40 |

Enfin, une première étude de répétabilité a été réalisée pour l'analyse du culot d'une culture de *M. intracellulare* contenant $5 \times 10^6$ bacilles.mL$^{-1}$. Un coefficient de variation de 94 % a été calculé pour 5 répétitions.

**2.9. Application de la méthode de détection électrochimique de l'invention au suivi de croissance de *Mycobacterium tuberculosis* H37Rv en milieu liquide**

2.9.1. Comparaison du délai de positivité d'une culture liquide de *Mycobacterium tuberculosis* H37Rv avec l'automate BD BACTEC™ MGIT™

Pour ce faire, trois volumes de milieu (volumes, A, B et C) ont été préparés pour tester la méthode de détection décrite dans l'invention (Tableau 3).

**Tableau 3** : Volumes des différents réactifs utilisés pour préparer les milieux de culture

| Réactifs | BACTEC™ | Détection électrochimique | | |
|---|---|---|---|---|
| | | Volume A | Volume B | Volume C |
| Milieu BD BACTEC™ MGIT™ | 7 ml | 3,5 ml | 2 ml | 1 ml |
| Supplément de croissance et BBL MGIT™ PANTA | 0,8 ml | 0,4 ml | 230 µl | 115 µl |
| p-AP-OG 2 x 10$^{-2}$ M | | 100 µl | 57 µl | 29 µl |
| Tréhalose 5 x 10$^{-1}$ M | | 100 µl | 57 µl | 29 µl |
| **Volume final** | **7,8 ml** | **4,1 ml** | **2,344 ml** | **1,173 ml** |

Une suspension bactérienne de *M. tuberculosis* a été préparée puis diluée deux fois au dixième dans du milieu de culture 7H9 (échantillons -1 et -2). Ces suspensions diluées ont servi à ensemencer les différents tubes.

Les tubes incubés dans l'automate BACTEC™ ont bénéficié d'une mesure automatique de la fluorescence une fois par heure. Le délai de positivité a été exprimé en jour.

Les tubes ont été analysés quotidiennement par la méthode de l'invention, en prélevant un volume de 30 μL de la culture et en le déposant à la surface d'un capteur sérigraphié sans traitement préalable. Les mesures ont été réalisées en voltammétrie linéaire et la positivité de l'échantillon correspond à l'apparition d'un pic d'oxydation de p-AP-G vers ~ +0.50 V *vs.* Ag/AgCl. Comme les mesures électrochimiques n'ont pas été réalisées en continu (une fois par jour dans le meilleur des cas), il existe pour l'instant une incertitude sur l'apparition du moment exact de la positivité d'où l'expression des résultats sous la forme ≤ x jours.

**Tableau 4** : Temps de positivité obtenus avec l'automate BACTEC™ et avec la méthode électrochimique ors de l'incubation de tubes témoins et de tubes inoculés avec *M. tuberculosis* H37Rv (1 à 2 répétitions par échantillon)

| Echantillons | BACTEC™ (délai en jours) | Détection électrochimique (délai en jours) | | |
|---|---|---|---|---|
| | | Volume A | Volume B | Volume C |
| Témoin 1 | négatif | négatif | négatif | négatif |
| Témoin 2 | négatif | négatif | | |
| -2 | **6,09** | ≤ **7** | ≤ **7** | ≤ **4** |
| -2 | **6,09** | ≤ **7** | ≤ **7** | ≤ **3** |
| -1 | **4,2** | ≤ **4** | ≤ **3** | ≤ **2** |
| -1 | **4,21** | ≤ **4** | ≤ **3** | ≤ **2** |

Les résultats du Tableau 4 indiquent que la méthode électrochimique a permis de faire un suivi de culture de *M. tuberculosis* dans des volumes plus faibles (Volume C) que la méthode BACTEC™, et ainsi, de réduire le temps de positivité de l'échantillon (≤ 2 jours au lieu de 4 jours pour la dilution -1 et ≤ 3-4 jours au lieu de 6 jours pour la dilution -2).

2.9.2. Comparaison du délai de positivité de quatre échantillons respiratoires inoculés ou non avec *M. tuberculosis* H37Rv, après fluidification-décontamination avec l'automate BD BACTEC™ MGIT™

Quatre échantillons respiratoires ont été fournis par le CHU de Dijon (Echantillon 1 et 2 : fibroscopie, Echantillon 3 : lavage broncho-alvéolaire et Echantillon 4 : crachat). Pour chaque échantillon, une aliquote de 3 ml a été contaminée avec 500 μl de suspension de *M. tuberculosis* H37Rv, une deuxième aliquote non contaminée a servi de témoin. Après une étape de fluidification-décontamination (Kit NacPac Biocentric), chaque culot a été remis en suspension dans 1,1 mL de milieu de culture. Ensuite, deux volumes de 500 μL de la solution précédente sont respectivement introduits dans un tube BACTEC™ et un tube Falcon contenant le milieu A (Tableau 3).

Les tubes incubés dans l'automate BACTEC™ ont bénéficié d'une mesure automatique de la fluorescence une fois par heure. Le délai de positivité a été exprimé en jour.

Les cultures réalisées dans les volumes de milieu A (Tableau 3) ont été incubées à 37°C et analysées avec la méthode électrochimique de l'invention. Les mesures électrochimiques et leur interprétation ont été réalisées comme dans le paragraphe 2.9.1. précédent.

**Tableau 5 :** Temps de positivité de quatre échantillons respiratoires contaminés ou non ensemencés (témoins) dans des tubes MGIT obtenus avec la méthode électrochimique et l'automate BACTEC

| Echantillons respiratoires | BACTEC™ (délai en jours) | | Détection électrochimique (délai en jours) | |
|---|---|---|---|---|
| | Témoin | Inoculé | Témoin | Inoculé |
| Echantillon 1 | - | **9,15** | - | ≤ **7** |
| Echantillon 2 | - | **4,16** | - | ≤ **5** |
| Echantillon 3 | - | **5,07** | - | ≤ **5** |
| Echantillon 4 | - | **5,20** | - | ≤ **5** |

Les résultats rassemblés dans le Tableau 5 montrent que la méthode électrochimique a été aussi performante que le BACTEC™ pour la détection en culture liquide d'échantillons respiratoires artificiellement contaminés par *M. tuberculosis.*

Enfin, en combinant les résultats des Tableaux 4 et 5, la méthode électrochimique de l'invention est capable de mettre en évidence plus rapidement la croissance de *M. tuberculosis* dans une culture liquide (~ 2 fois moins de temps) et ainsi sa présence dans un échantillon respiratoire.

**Liste de références**

1. Phunpae et al., Diagn. Microbiol. Infect. Dis., 78(3) : 242-248, 2014
2. Kashyap et al., BMC infectious diseases, 7 :74, 2007
3. Kashyap et al., Clin Diagn Lab Immunol., 12(6) :752-758, 2005
4. Elamin et al., J. Microbiol. Methods, 79(3) : 672-678, 2002
5. Boucau et al., Analytical Biochem., 385 : 120-127, 2009
6. Favrot et al., J. Biol. Chem., 289(36) : 25031-25040, 2014
7. Demande internationale WO 2011 /030160
8. Demande de Brevet CN102087283
9. Wiker et Harboe, Microbiol. Rev., 56(4) : 648-661, 1992
10. Sweeney et al., Lett. Appl. Microbiol., 43(4) :364-369, 2006
11. Sweeney et al., Appl. Environ. Microbiol., 73(22) : 7471-7473, 2007.

**Revendications**

1. Procédé de détection électrochimique de mycobactéries dans un échantillon biologique, ledit procédé comprenant les étapes de :

   a) sélection d'un substrat d'au moins une acyltransférase et de son cofacteur ;
   b) mise en contact dudit échantillon biologique avec lesdits substrat et cofacteur ;
   c) détection électrochimique du produit résultant de l'activité catalytique de ladite au moins une acyltransférase.

2. Procédé selon la revendication 1, où ladite au moins une acyltransférase est l'Antigène 85.

3. Procédé selon l'une quelconque des revendications 1 ou 2, où l'échantillon biologique est choisi parmi les cultures bactériennes, prélèvements biologiques d'origine humaine ou animale, et échantillons environnementaux.

4. Procédé selon l'une quelconque des revendications 1 à 3, dans lequel ledit substrat de ladite au moins une acyltransférase est choisi parmi le p-aminophényl-6-O-octanoyl-β-D-glucopyranoside, un substrat avec des groupements acyles possédant des chaînes alkyles allant de $C_7H_{15}$ à $C_{29}H_{59}$.

5. Procédé selon l'une quelconque des revendications 1 à 4, dans lequel ledit cofacteur est un sucre choisi dans le groupe constitué de tréhalose et D-glucose.

6. Procédé selon l'une quelconque des revendications 1 à 5, dans lequel l'étape de détection électrochimique c) est réalisée à l'aide d'un capteur ampérométrique.

7. Procédé selon l'une quelconque des revendications 1 à 6, où ledit échantillon biologique a été préalablement traité par un procédé d'isolement des mycobactéries comprenant les étapes de :

   A. mise en solution dudit échantillon biologique ;
   B. traitement avec un solvant apolaire de la solution obtenue à l'étape A);
   C. récupération des mycobactéries par filtration ou centrifugation de la solution issue de l'étape B) ; et
   D. récupération des mycobactéries à partir du filtrat ou du culot de centrifugation obtenu à l'issue de l'étape C).

8. Procédé selon la revendication 7, comprenant en outre une étape de décontamination A') de l'échantillon biologique mis en solution à l'issue de l'étape A) et avant l'étape B), et/ou une étape de décontamination C') de la membrane filtrante à l'issue de l'étape C) et avant l'étape D).

9. Procédé selon la revendication 8, où l'étape C') est réalisée avec des solutions acides et/ou basiques, et/ou ajout d'hypochlorite de sodium, et/ou ajout d'au moins un composé désinfectant.

10. Procédé selon la revendication 8 ou 9, comprenant en outre une étape de rinçage C") de la membrane filtrante à l'issue de l'étape C') et avant l'étape D).

11. Procédé selon la revendication 10, où l'étape de rinçage C") est réalisée avec un tampon phosphate.

12. Kit pour la mise en œuvre du procédé de détection électrochimique de mycobactéries dans un échantillon biologique tel que défini dans l'une quelconque des revendications 1 à 11 comprenant :

    i. un dispositif et les réactifs pour la collecte et la préparation de l'échantillon biologique à tester ;
    ii. un dispositif comprenant un substrat de l'Ag85 et son cofacteur pour l'incubation avec l'Ag85 ;
    iii. un dispositif pour la détection électrochimique à l'aide d'un lecteur adapté.

13. Kit selon la revendication 12, où le dispositif pour la détection électrochimique de l'étape iii) est un capteur ampérométrique, de préférence un capteur sérigraphié.

**Patentansprüche**

1. Verfahren zur elektrochemischen Detektion von Mykobakterien in einer biologischen Probe, wobei das Verfahren die folgenden Schritte umfasst:

    a) Auswahl eines Substrats für mindestens eine Acyltransferase sowie von deren co-Faktor;
    b) Inkontaktbringen der biologischen Probe mit diesem Substrat und diesem co-Faktor;
    c) elektrochemische Detektion des Produkts, welches infolge der katalytischen Aktivität der mindestens einen Acyltransferase entsteht.

2. Verfahren nach Anspruch 1, wobei es sich bei der mindestens einen Acyltransferase um das Antigen 85 handelt.

3. Verfahren nach einem beliebigen der Ansprüche 1 oder 2, wobei die biologische Probe aus den Bakterienkulturen, den biologischen Proben, welche vom Menschen oder von Tieren stammen, sowie aus Umweltproben ausgewählt ist.

4. Verfahren nach einem beliebigen der Ansprüche 1 bis 3, wobei das Substrat der mindestens einen Acyltransferase aus p-Aminophenyl-6-O-octanoyl-$\beta$-D-glucopyranosid, einem Substrat mit Acylgruppen, deren Alkylketten im Bereich von $C_7H_{15}$ bis $C_{29}H_{59}$ liegen, ausgewählt ist.

5. Verfahren nach einem beliebigen der Ansprüche 1 bis 4, wobei es sich bei dem co-Faktor um einen Zucker handelt, der aus der Gruppe ausgewählt ist, welche aus Trehalose und D-Glucose besteht.

6. Verfahren nach einem beliebigen der Ansprüche 1 bis 5, wobei der Schritt c) der elektrochemischen Detektion mit Hilfe eines amperometrischen Sensors durchgeführt wird.

7. Verfahren nach einem beliebigen der Ansprüche 1 bis 6, wobei die biologische Probe im Vorfeld mittels eines Verfahrens zur Isolierung von Mykobakterien behandelt wurde, welches die folgenden Schritte umfasst:

    A. Inlösungbringen der biologischen Probe;
    B. Behandeln der Lösung, welche in Schritt A) erhalten wurde, mit einem unpolaren Lösungsmittel;
    C. Gewinnen der Mykobakterien durch Filtration oder Zentrifugation der Lösung, welche nach Abschluss von Schritt B) erhalten wurde; und
    D. Gewinnen der Mykobakterien ausgehend von dem Filtrat oder dem Zentrifugationsbodensatz, das/der nach Abschluss des Schrittes C) erhalten wurde.

8. Verfahren nach Anspruch 7, wobei es darüber hinaus, nach Abschluss des Schrittes A) und vor dem Schritt B), einen Schritt A') der Dekontaminierung der in Lösung gebrachten biologischen Probe und/oder, nach Abschluss des Schrittes C) und vor dem Schritt D), einen Schritt C') der Dekontaminierung der Filtermembran umfasst.

9. Verfahren nach Anspruch 8, wobei der Schritt C') mit sauren und/oder basischen Lösungen und/oder einem Zusatz von Natriumhypochlorit und/oder einem Zusatz mindestens einer desinfizierenden Verbindung durchgeführt wird.

10. Verfahren nach Anspruch 8 oder 9, wobei es darüber hinaus, nach Abschluss des Schrittes C') und vor dem Schritt D), einen Schritt C") der Spülung der Filtermembran umfasst.

11. Verfahren nach Anspruch 10, wobei Spülschritt C") mit einem Phosphatpuffer durchgeführt wird.

12. Anwendungseinheit zur Durchführung des Verfahrens zur elektrochemischen Detektion von Mykobakterien in einer biologischen Probe gemäß der Begriffsbestimmung in einem beliebigen der Ansprüche 1 bis 11, wobei sie Folgendes umfasst:

i. eine Vorrichtung und die Reagenzien zum Gewinnen und Aufbereiten der zu prüfenden biologischen Probe;
ii. eine Vorrichtung, die ein Substrat von Ag85 und dessen co-Faktor umfasst, für die Inkubation mit dem Ag85;
iii. eine Vorrichtung für die elektrochemische Detektion mit Hilfe eines geeigneten Lesegeräts.

13. Anwendungseinheit nach Anspruch 12, wobei es sich bei der Vorrichtung zur elektrochemischen Detektion des Schrittes iii) um einen amperometrischen Sensor, vorzugsweise einen siebdruckbasierten Sensor, handelt.

**Claims**

1. Process for electrochemical detection of mycobacteria in a biological sample, said process comprising the steps of:

a) selecting a substrate of at least one acyltransferase and its cofactor;
b) bringing said biological sample into contact with said substrate and cofactor;
c) electrochemically detecting the product resulting from the catalytic activity of said at least one acyltransferase.

2. Process as claimed in claim 1, wherein said at least one acyltransferase is Antigen 85.

3. Process as claimed in either one of claims 1 and 2, wherein the biological sample is chosen from bacterial cultures, biological specimens of human or animal origin, and environmental samples.

4. Process as claimed in any one of claims 1 to 3, wherein said substrate of said at least one acyltransferase is chosen from p-aminophenyl-6-O-octanoyl-β-D-glucopyranoside, and a substrate with acyl groups having alkyl chains ranging from $C_7H_{15}$ to $C_{29}H_{59}$.

5. Process as claimed in any one of claims 1 to 4, wherein said cofactor is a sugar chosen from the group consisting of trehalose and D-glucose.

6. Process as claimed in any one of claims 1 to 5, wherein the electrochemical detection step c) is carried out by means of an amperometric sensor.

7. Process as claimed in any one of claims 1 to 6, wherein said biological sample has been treated beforehand by means of a process for isolating mycobacteria, comprising the steps of:

A. placing said biological sample in solution;
B. treating with an apolar solvent the solution obtained in step A);
C. recovering the mycobacteria by filtration or centrifugation of the solution resulting from step B); and
D. recovering the mycobacteria from the filtrate or from the centrifugation pellet obtained at the end of step C).

8. Process as claimed in claim 7, also comprising a step of decontaminating A') the biological sample placed in solution at the end of step A) and before step B), and/or a step of decontaminating C') the filtering membrane at the end of step C) and before step D).

9. Process as claimed in claim 8, wherein step C') is carried out with acid solutions and/or basic solutions, and/or addition of sodium hypochlorite, and/or addition of at least one disinfecting compound.

10. Process as claimed in claim 8 or 9, also comprising a step of rinsing C") the filtering membrane at the end of step C') and before step D).

**11.** Process as claimed in claim 10, wherein the rinsing step C") is carried out with a phosphate buffer.

**12.** Kit for carrying out the process for electrochemical detection of mycobacteria in a biological sample as defined in any one of claims 1 to 11, comprising:

   i. a device and the reagents for collecting and preparing the biological sample to be tested;
   ii. a device comprising a substrate of Ag85 and its cofactor for the incubation with Ag85;
   iii. a device for the electrochemical detection by means of a suitable reader.

**13.** Kit as claimed in claim 12, wherein the device for the electrochemical detection of step iii) is an amperometric sensor, preferably a screen-printed sensor.

FIGURE 1

FIGURE 2

FIGURE 3

FIGURE 4

FIGURE 5

**Etape 1** : Culture de
*Mycobacterium bovis* en
milieu liquide

Centrifugation 4 000 × g   6 minutes

**Etape 2** : Formation d'un
culot bactérien

Elimination du surnageant
Ajout de tampon phosphate pH 7,5 50 mM
Centrifugation 4 000 × g   6 minutes

**Etape 3** : Rinçage pour
éliminer les protéines
interférentes

Elimination du surnageant

**Etape 4** : Catalyse enzymatique

Ajout du substrat et du tréhalose

Incubation à 37°C pendant 24 heures

Récupération du produit par centrifugation à
14 000 × g   15 minutes

**Etape 5** : Récupération du produit
Mesure ampérométrique

Microcon
10 kD

FIGURE 6

FIGURE 7

A

B

C

FIGURE 8

A

B

C

FIGURE 9

FIGURE 10

FIGURE 11

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2011030160 A

- CN 102087283

**Littérature non-brevet citée dans la description**

- **PHUNPAE et al.** *Diagn. Microbiol. Infect. Dis.,* 2014, vol. 78 (3), 242-248
- **KASHYAP et al.** *BMC infectious diseases,* 2007, vol. 7, 74
- **KASHYAP et al.** *Clin Diagn Lab Immunol.,* 2005, vol. 12 (6), 752-758
- **ELAMIN et al.** *J Microbiol Methods,* 2002, vol. 79 (3), 672-678
- **BOUCAU et al.** *Analytical Biochem.,* 2009, vol. 385, 120-127
- **FAVROT et al.** *J. Biol. Chem.,* 2014, vol. 289 (36), 25031-25040
- **WIKER ; HARBOE.** *Microbiol. Rev.,* 1992, vol. 56 (4), 648-661

- **SWEENEY et al.** *Lett. Appl Microbiol.,* 2006, vol. 43 (4), 364-369
- **SWEENEY et al.** *Appl Environ Microbiol.,* 2007, vol. 73 (22), 7471-7473
- **KASHYAP et al.** *Clin Diagn Lab Immunol,* 2005, vol. 12 (6), 752-758
- **ELAMIN et al.** *J. Microbiol. Methods,* 2002, vol. 79 (3), 672-678
- **SWEENEY et al.** *Lett. Appl. Microbiol.,* 2006, vol. 43 (4), 364-369
- **SWEENEY et al.** *Appl. Environ. Microbiol.,* 2007, vol. 73 (22), 7471-7473